# EUROPEAN PATENT APPLICATION

(11) **EP 3 422 013 A1**
(43) Date of publication of application: **02.01.2019**
(21) Application number: 17744384.3
(22) Date of filing: 27.01.2017
(51) Int. Cl.: G01N 33/92, G01N 33/88, C12Q 1/02

(54) **FOOD ALLERGY BIOMARKER, FOOD ALLERGY TESTING METHOD, KIT FOR URINE SAMPLE TESTING, AND STICK FOR URINE SAMPLE TESTING**

(30) Priority: 29.01.2016 JP 2016015681
(71) Applicant: The University Of Tokyo, Tokyo 113-8654 (JP)
(72) Inventor: MURATA Takahisa, Tokyo 113-8654 (JP); NAKAMURA Tatsuro, Tokyo 113-8654 (JP); HAMABATA Taiki, Tokyo 113-8654 (JP)
(74) Representative: Desaix, Anne
(86) International application number: PCT/JP2017/002919
(87) International publication number: WO 2017/131154

(57) **Abstract**

This biomarker is a biomarker for testing a food allergy, and comprises at least one species selected from the group consisting of LTE₄, 14,15-LTE₄, 11-trans LTE₄, 11-dehydro 11-dehydro TXB₂, 2,3-dinor-8-iso Prostaglandin F_{2α}, 13,14-dihydro-15-keto-tetranor PGF_{1β}, 6,15-diketo-13,14-dihydro PGF_{1α}, tetranor-PGFM, 20-OH PGE₂, PGE₃, PGD₃, 13,14-dihydro-15-keto-tetranor PGD₂, and 13,14-dihydro-15-keto-tetranor PGE₂.

## Description

### Technical Field

The present invention relates to a food allergy biomarker, a food allergy testing method, a kit for testing a urine sample, and a stick for testing a urine sample.

Priority is claimed on Japanese Patent Application No. 2016-015681, filed January 29, 2016, the content of which is incorporated herein by reference.

### Background Art

Food allergies refer to a variety of allergic reactions occurring by introducing allergens contained in food to the body. The symptoms of an allergy include diarrhea, vomiting, dermatitis, etc., and when the allergy becomes severe, a shock occurs, leading to death. In Japan, the adult morbidity rate is as high as 2.6%, and the neonatal morbidity rate is higher, which is 5.6%, and many severe cases have been reported. For this reason, food allergies have become a big issue.

Other than eggs, milk and wheat, which are well known as allergens, there are many kinds of food that can serve as allergens, which cause strong symptoms only with a very small amount. That's why a food allergy is dangerous. Although there are no other ways to prevent a food allergy except the identification of allergens and avoidance of eating them, under the current food situation in which processed food is prevalent worldwide, it is difficult to identify allergens and avoid eating them, and the quality of life (QOL) of patients who cannot eat food in peace is significantly declined. In addition, particularly in the case of children, the elimination of causative substances may produce an adverse effect on nutrition. Further, due to a recent change in living environment, the balance of immune responses in the body is impaired, resulting in an additional increase in food allergy morbidity or the aggravation of symptoms. Because of these reasons, there are urgent demands for development of drugs for treating a food allergy, and establishment of early diagnostic technology or experimental systems for evaluating food allergenicity.

The mechanism of developing a food allergy has been known as follows:
i) the production of IgE by the reaction between T and B cells and allergens entering into the body; and
ii) when the allergens enter again into the body, the secretion (degranulation) of active substances such as histamine from IgE-binding mast cells and the occurrence of severe inflammation.

In addition, as one of the method for treating a food allergy, hyposensitization therapy may be used. The hyposensitization therapy is for inducing immune tolerance by administering a diluted allergen to a patient under the supervision of a doctor. While many methods for treating allergic diseases are symptomatic therapies, the hyposensitization therapy has attracted attention because it acts on the action mechanism of an allergic disease and aims at radical curing of the disease. The hyposensitization therapy is started with a very low dose and carried out by gradually increasing the dose.

Currently, as a method for diagnosing a food allergy, a diagnostic method including measurement of blood IgE is used (e.g., see Non-Patent Document 1).

### Prior Art Documents

### Non Patent Documents

Non Patent Document 1: Guidance of medical care of food allergy by the Health, Labor and Welfare Science Research Group 2011 (http://www.allergy.go.jp/allergy/guideline/05/05_2011.pdf)

### DISCLOSURE OF THE INVENTION

### Problems to be Solved by the Invention

For hyposensitization therapy, when an allergen dose gradually increases, in order to prevent severe symptoms caused by administrating an allergen at a dose higher than the threshold value, the diagnosis is carried out according to the visual judgment of a doctor. However, such judgment is just an arbitrary determination, not a quantitative diagnosis. Therefore, a means for suitably evaluating the severity of allergic symptoms in a patient is required.

In addition, in the diagnostic method of measuring blood IgE, the presence or absence of allergic reactions may be expected, but the risk of or severity of symptoms may not be known. In other words, the amount of an IgE antibody in the blood does not often correspond to the risk or severity of food allergic symptoms. In addition, since this method needs blood collection, it should be carried out in medical instruments, and blood collection itself is also a burden on infants.

The present invention is deduced by taking into account of the above-mentioned circumstances, and thus provides a testing method of simply and suitably evaluating the risk of developing a food allergy, the severity of symptoms thereof and prognosis thereof.

### Means for Solving the Problems

To attain the above-mentioned object, the inventors focused on the fact that a metabolite of a physiologically active substance produced in mast cells is excreted into urine since the occurrence and progression of a food allergy are very highly dependent on mast cells and thus can be stably detected, and conducted a comprehensive concentration analysis of lipids in urine samples. As a result, they found a lipid whose content varies from a urine sample according to the expression of symptoms of a food allergy, and therefore the present invention was completed.

That is, the present invention includes the following aspects.
[1] A food allergy biomarker, which includes
   at least one selected from the group consisting of Leukotriene E₄ (LTE₄), 14,15-LTE₄, 11-trans LTE₄, 11-dehydro Thromboxane B₂ (11-dehydro TXB₂), 2,3-dinor-8-iso Prostaglandin F_{2α}, 13,14-dihydro-15-keto-tetranor PGF_{1β}, 6,15-diketo-13,14-dihydro PGF_{1α}, tetranor-Prostaglandin F Metabolite (tetranor-PGFM), 20-hydroxy Prostaglandin E₂ (20-OH PGE₂), PGE₃, Prostaglandin D₃ (PGD₃), 13,14-dihydro-15-keto-tetranor PGD₂, and 13,14-dihydro-15-keto-tetranor PGE₂.
[2] A food allergy testing method, which includes a measurement step of measuring the content of a biomarker in a urine sample of a subject, wherein the biomarker is at least one selected from the group consisting of LTE₄, 14,15-LTE₄, 11-trans LTE₄, 11-dehydro TXB₂, 2,3-dinor-8-iso Prostaglandin F_{2α}, 13,14-dihydro-15-keto-tetranor PGFip, 6,15-diketo-13,14-dihydro PGF_{1α}, tetranor-PGFM, 20-OH PGE₂, PGE₃, PGD₃, 13,14-dihydro-15-keto-tetranor PGD₂, and 13,14-dihydro-15-keto-tetranor PGE₂.
[3] The method described in [2], which further includes, in the measurement step, measuring a tetranor-PGDM or tetranor-PGEM content.
[4] The method described in [2] or [3], which further includes an evaluation step of evaluating whether, as a urine sample contains a high or low biomarker content, symptoms of a food allergy are severe or more severe, or the risk of developing a food allergy is high or higher.
[5] The method described in any one of [2] to [4], which is used to evaluate a method or drug for treating a food allergy.
[6] The method described in [5], which is used in hyposensitization therapy.
[7] The method described in any one of [2] to [6], wherein the measurement step is performed by an immunoassay or mass spectrometry.
[8] The method described in [7], which further includes a pretreatment step of adding at least one selected from the group consisting of deuterated LTC₄, deuterated 6-keto-PGF_{1α}, deuterated tetranor-PGDM, deuterated tetranor-PGEM, and deuterated PGE₂ as an internal standard to urine samples, and wherein the measurement step is performed by mass spectrometry.
[9] The method described in any one of [2] to [8], which further includes, in the evaluating step, evaluating the activation of mast cells involved in a food allergy.
[10] A kit for testing a urine sample for a food allergy, which includes at least one selected from the group consisting of an anti-LTE₄ antibody, an anti-14,15-LTE₄ antibody, an anti-11-trans LTE₄ antibody, an anti-11-dehydro TXB₂ antibody, an anti-2,3-dinor-8-iso PGF_{2α} antibody, an anti-13,14-dihydro-15-keto-tetranor PGFip antibody, an anti-6,15-diketo-13,14-dihydro PGF_{1α} antibody, an anti-tetranor-PGFM antibody, an anti-20-OH PGE₂ antibody, an anti-PGE₃ antibody, an anti-PGD₃ antibody, an anti-13,14-dihydro-15-keto-tetranor PGD₂ antibody, and an anti-13,14-dihydro-15-keto-tetranor PGE₂ antibody.
[11] The kit for testing a urine sample for a food allergy described in [10], which further includes an anti-tetranor-PGDM antibody or an anti-tetranor-PGEM antibody.
[12] The kit for testing a urine sample for a food allergy described in [10] or [11], which further includes at least one selected from the group consisting of deuterated LTC₄, deuterated 6-keto-PGF_{1α}, deuterated tetranor-PGDM, deuterated tetranor-PGEM, and deuterated PGE₂.
[13] The kit for testing a urine sample for a food allergy described in any one of [10] to [12], which further includes deuterated tetranor-PGDM.
[14] A stick for testing a urine sample for a food allergy, which includes at least one selected from the group consisting of an anti-LTE₄ antibody, an anti-14,15-LTE₄ antibody, an anti-11-trans LTE₄ antibody, an anti-11-dehydro TXB₂ antibody, an anti-2,3-dinor-8-iso PGF_{2α} antibody, an anti-13,14-dihydro-15-keto-tetranor PGFip antibody, an anti-6,15-diketo-13,14-dihydro PGF_{1α} antibody, an anti-tetranor-PGFM antibody, an anti-20-OH PGE₂ antibody, an anti-PGE₃ antibody, an anti-PGD₃ antibody, an anti-13,14-dihydro-15-keto-tetranor PGD₂ antibody, and an anti-13,14-dihydro-15-keto-tetranor PGE₂ antibody.
[15] The stick for testing a urine sample to detect a food allergy described in [14], which further includes an anti-tetranor-PGDM antibody or an anti-tetranor-PGEM antibody.

### Effects of the Invention

According to the present invention, the presence or absence of a food allergy, the risk of developing a food allergy and severity can be suitably evaluated. In addition, before allergic symptoms are shown, preventive treatment can be performed through risk evaluation. In addition, through the evaluation of the responsiveness to hyposensitization therapy, effective treatment can be carried out by administering an allergen as much as possible within a safe range.

In addition, the testing method of the present invention does not require medical techniques such as blood collection, and thus testing can be performed simply at home with respect to children through the elderly using a kit for testing a urine sample or stick for testing a urine sample according to the present invention.

### BRIEF DESCRIPTION OF THE DRAWINGS

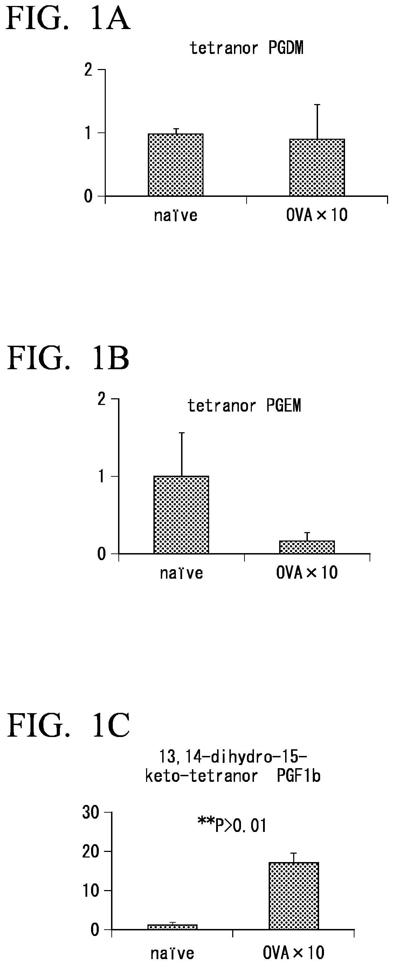

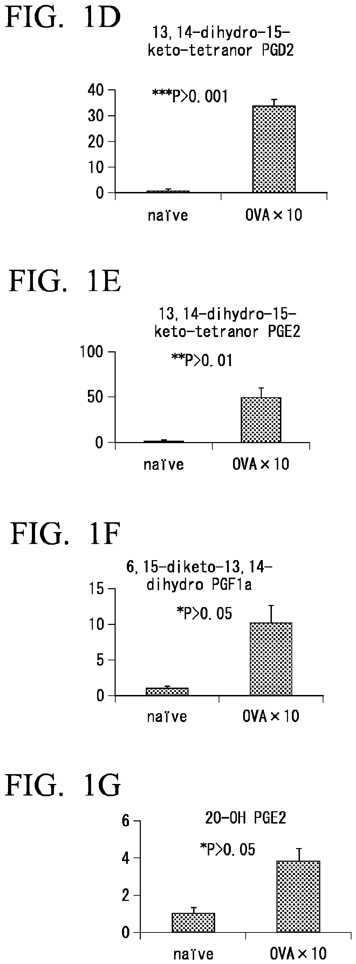

FIG. 1A is a graph showing the relative value of a tetranor PGDM content in a urine sample of a mouse model with food allergy when a tetranor PGDM content in a urine sample of an untreated mouse was set to be 1 according to Example 1.
FIG. 1B is a graph showing the relative value of a tetranor PGEM content in a urine sample of a mouse model with food allergy when a tetranor PGEM content in a urine sample of an untreated mouse was set to be 1 according to Example 1.
FIG. 1C is a graph showing the relative value of a 13,14-dihydro-15-keto-tetranor PGFip content in a urine sample of a mouse model with food allergy when a 13,14-dihydro-15-keto-tetranor PGFip content in a urine sample of an untreated mouse was set to be 1 according to Example 1.
FIG. 1D is a graph showing the relative value of a 13,14-dihydro-15-keto-tetranor PGD₂ content in a urine sample of a mouse model with food allergy when a 13,14-dihydro-15-keto-tetranor PGD₂ content in a urine sample of an untreated mouse was set to be 1 according to Example 1.
FIG. IE is a graph showing the relative value of a 13,14-dihydro-15-keto-tetranor PGE₂ content in a urine sample of a mouse model with food allergy when a 13,14-dihydro-15-keto-tetranor PGE₂ content in a urine sample of an untreated mouse was set to be 1 according to Example 1.
FIG. IF is a graph showing the relative value of a 6,15-diketo-13,14-dihydro PGF_{1α} content in a urine sample of a mouse model with food allergy when a 6,15-diketo-13,14-dihydro PGF_{1α} content in a urine sample of an untreated mouse was set to be 1 according to Example 1.
FIG. 1G is a graph showing the relative value of a 20-OH PGE₂ content in a urine sample of a mouse model with food allergy when a 20-OH PGE₂ content in a urine sample of an untreated mouse was set to be 1 according to Example 1.

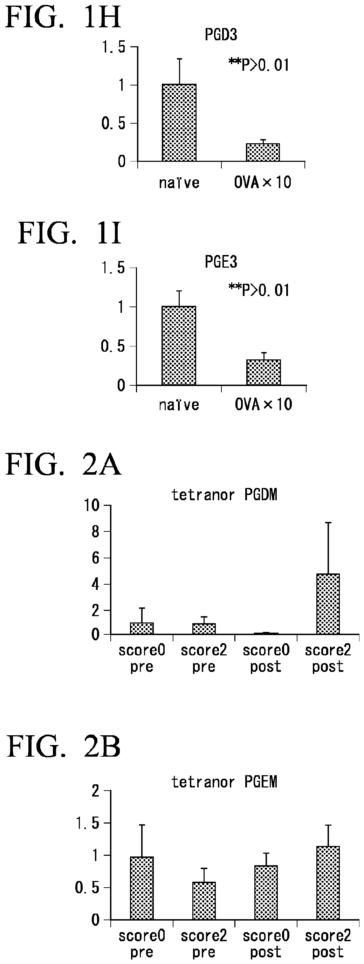

FIG. 1H is a graph showing the relative value of a PGD₃ content in a urine sample of a mouse model with food allergy when a PGD₃ content in a urine sample of an untreated mouse was set to be 1 according to Example 1.
FIG. 1I is a graph showing the relative value of a PGE₃ content in a urine sample of a mouse model with food allergy when a PGE₃ content in a urine sample of an untreated mouse was set to be 1 according to Example 1.
FIG. 2A is a graph showing the relative values of tetranor PGDM contents in urine samples of a patient (human) with a score of 0 after a food allergy intolerance test and a patient (human) with a score of 2 before and after the food allergy intolerance test, when a tetranor PGDM content in a urine sample of a patient (human) with a score of 0 before the food allergy intolerance test was set to be 1 according to Example 2.
FIG. 2B is a graph showing the relative values of tetranor PGEM contents in urine samples of a patient (human) with a score of 0 after a food allergy intolerance test and a patient (human) with a score of 2 before and after the food allergy intolerance test, when a tetranor PGEM content in a urine sample of a patient (human) with a score of 0 before the food allergy intolerance test was set to be 1 according to Example 2.

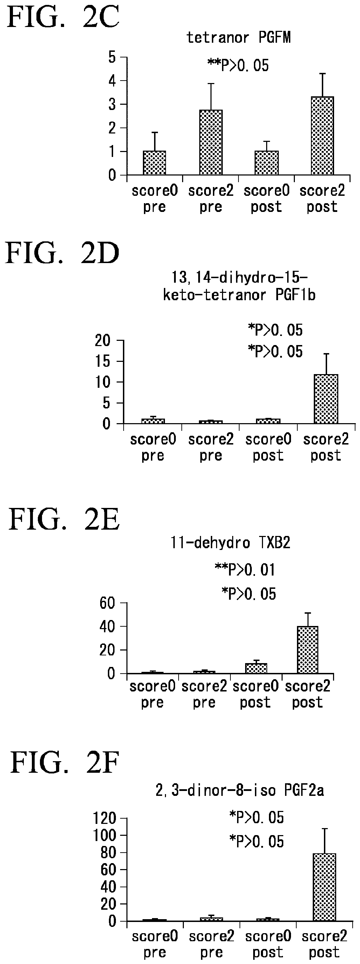

FIG. 2C is a graph showing the relative values of tetranor PGFM contents in urine samples of a patient (human) with a score of 0 after a food allergy intolerance test and a patient (human) with a score of 2 before and after the food allergy intolerance test, when a tetranor PGFM content in a urine sample of a patient (human) with a score of 0 before the food allergy intolerance test was set to be 1 according to Example 2.
FIG. 2D is a graph showing the relative values of 13,14-dihydro-15-keto-tetranor PGFip contents in urine samples of a patient (human) with a score of 0 after a food allergy intolerance test and a patient (human) with a score of 2 before and after the food allergy intolerance test, when a 13,14-dihydro-15-keto-tetranor PGFip content in a urine sample of a patient (human) with a score of 0 before the food allergy intolerance test was set to be 1 according to Example 2.
FIG. 2E is a graph showing the relative values of 11-dehydro TXB₂ contents in urine samples of a patient (human) with a score of 0 after a food allergy intolerance test and a patient (human) with a score of 2 before and after the food allergy intolerance test, when a 11-dehydro TXB₂ content in a urine sample of a patient (human) with a score of 0 before the food allergy intolerance test was set to be 1 according to Example 2.
FIG. 2F is a graph showing the relative values of 2,3-dinor-8-iso PGF_{2α} contents in urine samples of a patient (human) with a score of 0 after a food allergy intolerance test and a patient (human) with a score of 2 before and after the food allergy intolerance test, when a 2,3-dinor-8-iso PGF_{2α} content in a urine sample of a patient (human) with a score of 0 before the food allergy intolerance test was set to be 1 according to Example 2.

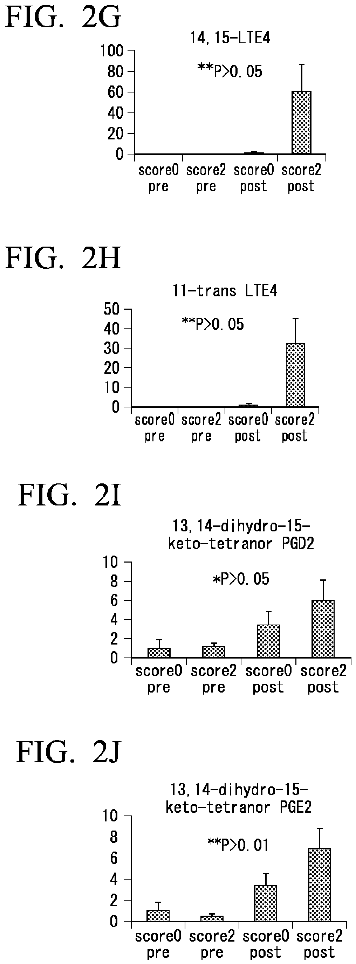

FIG. 2G is a graph showing the relative values of 14,15-LTE₄ contents in urine samples of a patient (human) with a score of 0 after a food allergy intolerance test and a patient (human) with a score of 2 before and after the food allergy intolerance test, when a 14,15-LTE₄ content in a urine sample of a patient (human) with a score of 0 before the food allergy intolerance test was set to be 1 according to Example 2.
FIG. 2H is a graph showing the relative values of 11-trans LTE₄ contents in urine samples of a patient (human) with a score of 0 after a food allergy intolerance test and a patient (human) with a score of 2 before and after the food allergy intolerance test, when a 11-trans LTE₄ content in a urine sample of a patient (human) with a score of 0 before the food allergy intolerance test was set to be 1 according to Example 2.
FIG. 2I is a graph showing the relative values of 13,14-dihydro-15-keto-tetranor PGD₂ contents in urine samples of a patient (human) with a score of 0 after a food allergy intolerance test and a patient (human) with a score of 2 before and after the food allergy intolerance test, when a 13,14-dihydro-15-keto-tetranor PGD₂ content in a urine sample of a patient (human) with a score of 0 before the food allergy intolerance test was set to be 1 according to Example 2.
FIG. 2J is a graph showing the relative values of 13,14-dihydro-15-keto-tetranor PGE₂ contents in urine samples of a patient (human) with a score of 0 after a food allergy intolerance test and a patient (human) with a score of 2 before and after the food allergy intolerance test, when a 13,14-dihydro-15-keto-tetranor PGE₂ content in a urine sample of a patient (human) with a score of 0 before the food allergy intolerance test was set to be 1 according to Example 2.

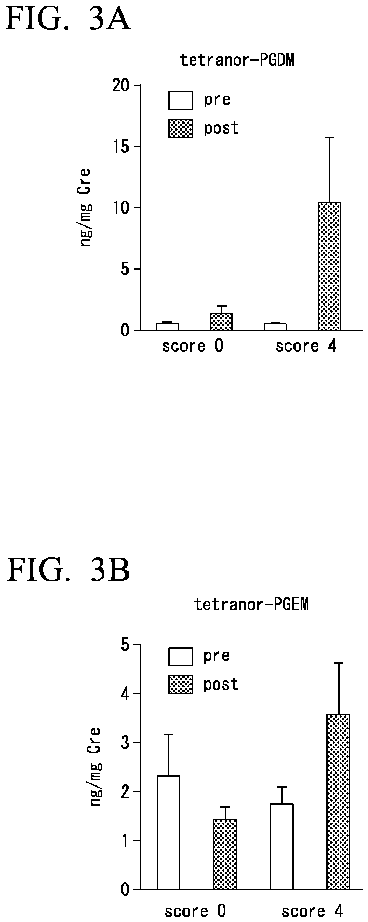

FIG. 3A is a graph showing tetranor PGDM contents (absolute values) in urine samples of patients (human) with scores of 0 and 4 before and after a food allergy intolerance test according to Example 3.
FIG. 3B is a graph showing tetranor PGEM contents (absolute values) in urine samples of patients (human) with scores of 0 and 4 before and after a food allergy intolerance test according to Example 3.

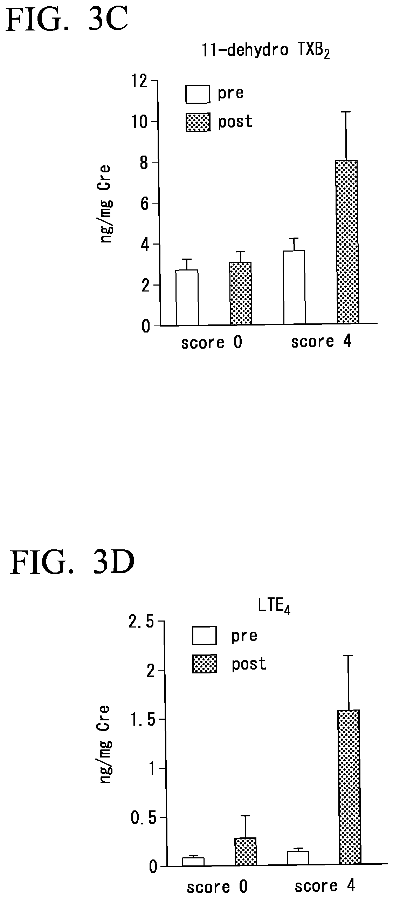

FIG. 3C is a graph showing 11-dehydro TXB₂ contents (absolute values) in urine samples of patients (human) with scores of 0 and 4 before and after a food allergy intolerance test according to Example 3.
FIG. 3D is a graph showing LTE₄ contents (absolute values) in urine samples of patients (human) with scores of 0 and 4 before and after a food allergy intolerance test according to Example 3.

### BEST MODE FOR CARRYING OUT THE INVENTION

### <Food allergy biomarker>

In one exemplary embodiment, the present invention provides a biomarker for detecting a food allergy, which consists of at least one selected from the group consisting of Leukotriene E₄ (LTE₄), 14,15-LTE₄, 11-trans LTE₄, 11-dehydro Thromboxane B₂ (11-dehydro TXB₂), 2,3-dinor-8-iso Prostaglandin F_{2α}, 13,14-dihydro-15-keto-tetranor PGF_{1β}, 6,15-diketo-13,14-dihydro PGF_{1α}, tetranor-Prostaglandin F Metabolite (tetranor-PGFM), 20-hydroxy Prostaglandin E₂ (20-OH PGE₂), PGE₃, Prostaglandin D₃ (PGD₃), 13,14-dihydro-15-keto-tetranor PGD₂, and 13,14-dihydro-15-keto-tetranor PGE₂.

According to the biomarker of the exemplary embodiment, it is possible to suitably evaluate not only the presence or absence of a food allergy, but also the risk and severity of symptoms.

The "food allergy" used herein refers to a variety of allergic reactions caused by introducing an allergen contained in food into the body. Food allergies may be caused by, for example, eggs, milk, crustaceans, wheat, fruits, nuts, fish and shellfish and buckwheat, or allergens other than these. Allergic symptoms appear on, for example, the skin, mucosa, digestive organs and respiratory organs. Examples of typical symptoms include diarrhea, vomiting and dermatitis.

The "food allergy biomarker" used herein refers to a substance whose amount serves as an indicator of the risk of developing a food allergy, the severity of symptoms of the allergy, and prognosis.

In terms of the biomarker of the exemplary embodiment, the names, structural formulas, and brief descriptions of specific lipids, and an increase/decrease in content in a urine sample according to the expression of symptoms of a food allergy are shown in the following table.

**[Table 1] s**

| Name of lipid | Structural Formula | Brief description | Change in content of urine sample according to expression of symptoms of food allergy (increase/decrease) |
|---|---|---|---|
| LTE₄ | (1) | Produced by the action of | Increase |
| 14,15-LTE₄ | (2) | Metabolite of 14,15-LTC₄ | Increase |
| 11-trans LTE₄ | (3) | Isomer of LTE₄ | Increase |
| 11-dehydro TXB₂ | (4) | Metabolite of TXB₂ | Increase |
| 2,3-dinor-8-iso PGF_{2α} | (5) | Metabolite of 8-iso PGF_{2α} | Increase |
| 13,14-dihydro-15-keto-tetranor PGF_{1β} | (6) | Metabolite of PGE₂ | Increase |
| 6,15-diketo-13,14-dihydro PGF_{1α} | (7) | Metabolite of PGI₂ | Increase |
| Tetranor-PGFM | (8) | Metabolite of PGF_{2α} | Increase |
| 20-OH PGE₂ | (9) | Metabolite of PGE₂ | Increase |
| PGE₃ | (10) | Metabolite of EPA in COX pathway | Decrease |
| PGD₃ | (11) | Metabolite of EPA in COX pathway | Decrease |
| 13,14-dihydro-15-keto-tetranor PGD₂ | (12) | Metabolite of PGD₂ | Increase |
| 13,14-dihydro-15-keto-tetranor PGE₂ | (13) | Metabolite of PGE₂ | Increase |

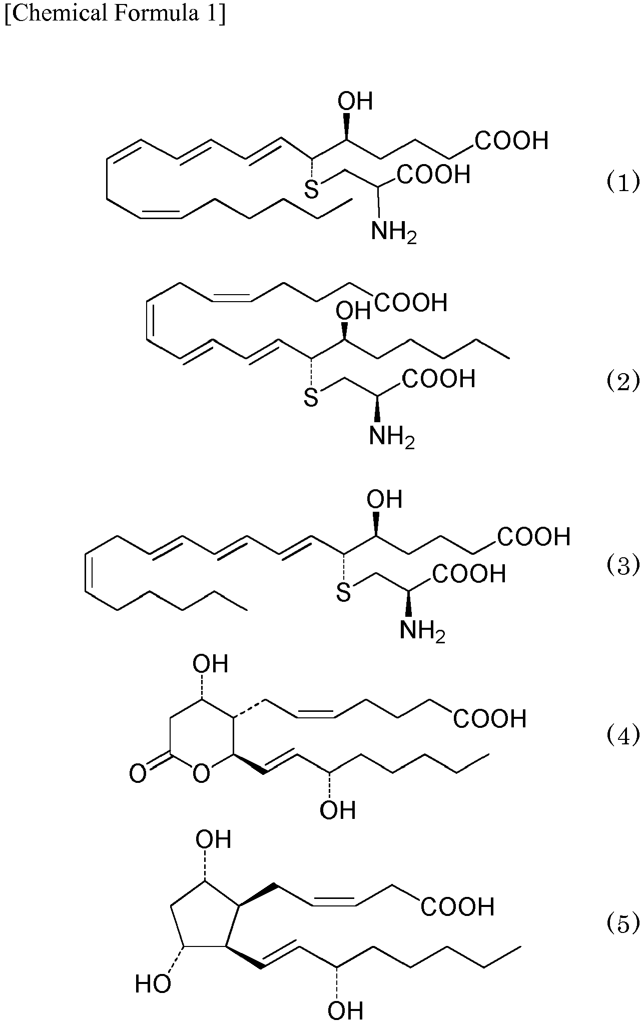

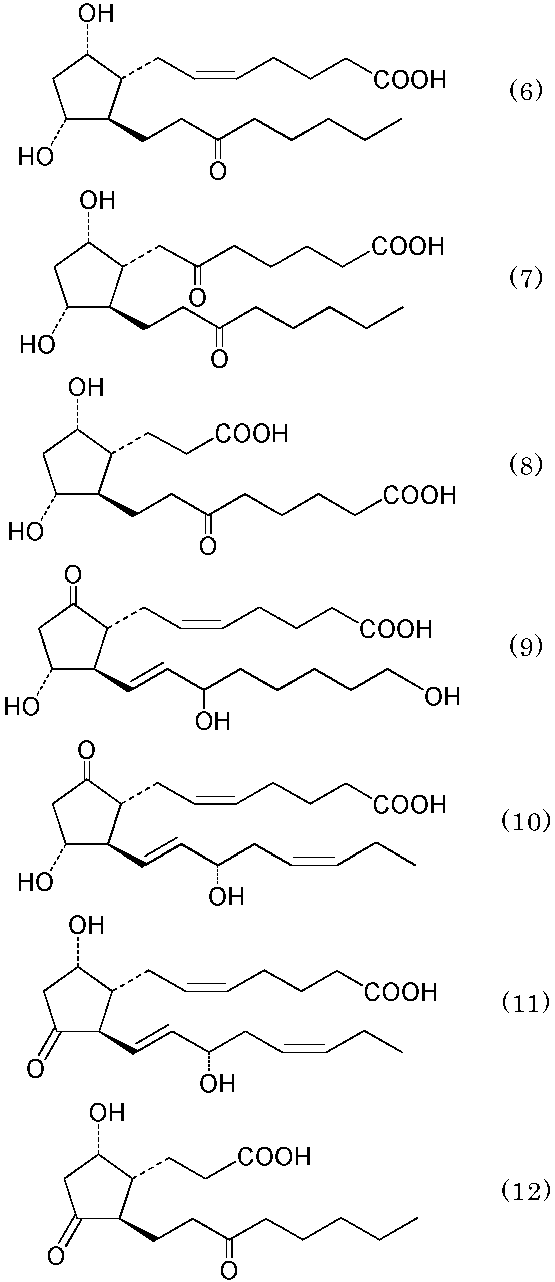

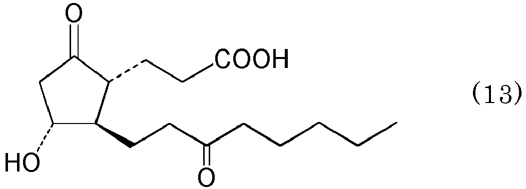

As shown in the exemplary embodiment, in food allergy patients, the contents of the above-described 13 types of lipids in urine samples are increased or decreased for a specific period, and therefore the lipids may be determined as food. In addition, the severity of symptoms may be evaluated from an increased or decreased value of a lipid content in a urine sample.

The "specific period" used herein refers to, for example, 3, 5, 7, 10 or 20 days or more after the intake of a causative substance of a food allergy.

In addition, among the above-described 13 types of lipids, LTE₄, 11-dehydro TXB₂, tetranor-PGFM, or 13,14-dihydro-15-keto-tetranor PGD₂ is preferable, and 11-dehydro TXB₂ or tetranor-PGFM is more preferable.

The amounts of the lipids are considered to increase or decrease in a mast cell-dependent manner, and it is known that an excessive amount of mast cells is present in the mucosa of the gastrointestinal tract where a food allergy occurs. Therefore, the presence or absence of a food allergy, the risk of developing a food allergy and the severity of symptoms may be suitably evaluated by examining a change in the amount of the lipid described above in blood, urine or feces collected from a subject after food is ingested and then digested. Among the samples, urine is preferably used in measurement of the above-described biomarker because it can be collected non-invasively and contains a large amount of the biomarker.

### <Food allergy testing method>

In one exemplary embodiment, the present invention provides a food allergy testing method, which includes a step of measuring a biomarker content in a urine sample of a subject, wherein the biomarker is at least one selected from the group consisting of LTE₄, 14,15-LTE₄, 11-trans LTE₄, 11-dehydro TXB₂, 2,3-dinor-8-iso Prostaglandin F_{2α}, 13,14-dihydro-15-keto-tetranor PGF_{1β}, 6,15-diketo-13,14-dihydro PGF_{1α}, tetranor-PGFM, 20-OH PGE₂, PGE₃, PGD₃, 13,14-dihydro-15-keto-tetranor PGD₂, and 13,14-dihydro-15-keto-tetranor PGE₂.

According to the testing method of the exemplary embodiment, as well as the presence or absence of a food allergy, the risk of developing a food allergy and the severity of symptoms may be suitably evaluated. In addition, before the expression of allergic symptoms, preventive treatment may be performed by evaluating the risk.

The testing method according to the embodiment does not require a medical technique such as blood collection, and may be easily conducted at home with respect to children through the elderly using a kit for testing a urine sample or a stick for testing a urine sample, which will be described below.

The "subject" used herein refers to a person who is likely to have a food allergy, a person who actually has a food allergy, a person who is receiving treatment for a food allergy, a person who is taking a drug for treating food allergy, or a person who is uncertain to have a food allergy. In addition, the "subject" is not limited to a human, and thus may be a mammal such as a mouse, a rat, a rabbit, a cat, a dog, a monkey, a pig, sheep, a cow, a horse or the like.

### [Measurement step]

In the measurement step, urine collected from a subject is used as a sample. The urine used in the testing method of the exemplary embodiment may be collected according to a conventional method. The urine may be a single collection of urine, or twice or more collections of urine. The urine sample may be stored at room temperature before measurement, or may be stored at -40°C or less, for example, -80°C. The frozen urine may be rapidly thawed until used in measurement.

In addition, as described in <Food allergy biomarker>, the biomarker is contained in the blood, urine or feces of a subject, but the urine is preferably used because it can be non-invasively collected and contains a large amount of the biomarker.

A method of measuring a content of the above-described biomarker in a urine sample may be performed using any method of detecting or measuring a specific substance in a solution. The "method of measuring a content of a biomarker in a urine sample" used herein includes not only accurately measuring an amount of the biomarker, but also detecting the presence or absence of the biomarker, or determining whether the amount of the biomarker is larger or smaller than a specific amount. The content of the above-described biomarker in a urine sample may be corrected according to the amount of a reference substance such as creatinine.

In addition, it is preferable to measure a tetranor PGDM or tetranor PGEM content in addition to at least one of the above-described 13 types of lipids, and it is more preferable to measure tetranor PGDM and tetranor PGEM contents.

The tetranor PGDM or tetranor PGEM was found, by the inventors, as a food allergy biomarker, which is disclosed in WO/2016/021704.

Although details of mechanisms of producing and metabolizing a lipid are uncertain because there are many types of lipids, it is considered that PGD₂, TXB₂, and the metabolites thereof (e.g., tetranor PGDM and 11-dehydro TXB₂) are derived from mast cells, and the lipids among the above-described 13 types of lipids, other than the metabolites of PGD₂ and TXB₂, are considered to be derived from mast cells or products of a secondary inflammatory reaction occurring by the activation thereof.

Therefore, by measuring the tetranor PGDM or tetranor PGEM content in addition to the above-described 13 types of lipids, a plurality of lipids can be used as an indicator, and thus it is possible to determine the presence or absence of a food allergy, the risk of developing a food allergy and severity with a higher accuracy.

As a method of measuring the content of the above-described biomarker in a urine sample, for example, immunoassay, agglutination, turbidimetry, western blotting, a surface plasmon resonance (SPR) method, various types of chromatography techniques (e.g., gas chromatography, liquid chromatography, high performance liquid chromatography, etc.), mass spectrometry, etc. may be used, but the present invention is not limited thereto. Among these methods, immunoassay or mass spectrometry for measuring a content of the above-described biomarker in a urine sample using an antigen-antibody reaction of an antibody specifically binding to the above-described biomarker and the above-described biomarker in a urine sample is preferably used.

### (Immunoassay)

In immunoassay, a detectably-labeled antibody specifically binding to the above-described biomarker, or a detectably-labeled antibody (secondary antibody) against an antibody specifically binding to any one of the above-described biomarkers is used. As a method of labeling an antibody, for example, enzyme immunoassay (EIA) or enzyme-linked immunosorbent assay) (ELISA), radioimmunoassay (RIA), fluoroimmunoassay (FIA), fluorescence polarization immunoassay (FPIA), or chemiluminescent immunoassay (CLIA) may be used.

In ELISA, an antibody labeled with an enzyme such as a peroxidase or an alkaline phosphatase is used, and in RIA, an antibody labeled with a radioactive substance such as ¹²⁵I, ¹³¹I, ³⁵S, or ³H is used, in FPIA, an antibody labeled with a fluorescent substance such as fluorescein isothiocyanate, rhodamine, dansyl chloride, phycoerythrin, tetramethylrhodamine isothiocyanate, or a near-infrared fluorescent substance is used, and in CLIA, an antibody labeled with a luminescent substance such as luciferase, luciferin or aequorin is used. Other than these, an antibody labeled with nanoparticles such as gold colloids or quantum dots may be used.

In the immunoassay, detection may be performed in such a manner that avidin or streptoavidin labeled with an enzyme or the like binds to a biotin-labeled antibody specifically binding to the above-described biomarker.

Among the kinds of immunoassays, ELISA using an enzyme label may simply and rapidly detect an antigen. ELISA includes a competitive method and a sandwich method.

In the competitive method, an antigen-antibody reaction is induced by immobilizing, among the above-described biomarkers, an antibody specifically binding to LTE₄ (hereinafter, called "anti-LTE₄ antibody") to a solid support such as a microplate, and adding a urine sample and enzyme-labeled LTE₄ that reacts with the antibody as an enzyme-labeled antigen. After washing, the support is reacted with an enzyme substrate and developed to measure an absorbance. The higher the LTE₄ content in the urine sample, the weaker the luminescence, and the lower the LTE₄ content in the urine sample, the stronger the luminescence. Using a calibration curve, the LTE₄ content in a urine sample can be obtained. When a biomarker other than LTE₄ is used, the content can be obtained in the same manner as described above.

In the sandwich method, for example, an anti-LTE₄ antibody is immobilized onto a solid support, a urine sample is added to react with the antibody, and then an anti-LTE₄ antibody that recognizes a different epitope of the enzyme-labeled antigen (LTE₄) is added to react with the previous antibody. After washing, the support may be reacted with an enzyme substrate and developed to measure an absorbance, thereby obtaining an LTE₄ content in a urine sample. When a biomarker other than LTE₄ is used, the content may be obtained in the same manner as described above.

Alternatively, in the sandwich method, an anti-LTE₄ antibody immobilized onto the solid support reacts with an antigen (LTE₄) in a urine sample, a non-labeled anti-LTE₄ antibody that recognizes a different epitope of the same antigen (LTE₄) is added, and an antibody (secondary antibody) labeled with an enzyme against the non-labeled anti-LTE₄ antibody may be further added.

When the enzyme is a peroxidase, the enzyme substrate may be 3,3'-diaminobenzidine (DAB), 3,3',5,5'-tetramethylbenzidine (TMB), or o-phenylenediamine (OPD), and when the enzyme is an alkaline phosphatase, the enzyme substrate may be a p-nitropheny phosphase (NPP).

The "solid support" used herein may be any support to which an antibody can be immobilized without particular limitation. Examples of the solid support may include, a microtiter plate, a substrate, beads, and a membrane according to its morphology, and examples of substances for the solid support may include inorganic substances such as silica, alumina, glass and metal, and organic polymers such as thermoplastic resins (e.g., nylon, polyvinylidene difluoride (PVDF), etc.), and nitrocellulose. A labeling substance or antibody may be immobilized onto the above-described solid support according to a known method.

An antibody specifically binding to any one of the above-described biomarkers may not only be a monoclonal antibody, but also a polyclonal antibody, and any antibody can be prepared according to a known method. The monoclonal antibody can be obtained by, for example, preparing a hybridoma by isolating antibody-producing cells from a non-human mammal immunized with any one of the above-described biomarkers and fusing the isolated cells with myeloma cells, and purifying an antibody produced by the hybridoma. In addition, the polyclonal antibody can be obtained from, for example, the serum of a non-human mammal immunized with any one of the above-described biomarkers.

### (Mass spectrometry)

When mass spectrometry is used in the measurement step, an isolation step for isolating a metabolite contained in a urine sample may be included before the measurement step. In the isolation step, time-resolved isolation of a metabolite in a urine sample is achieved. For isolation, any kind of chromatographic isolation technique may be used. Specifically, for example, liquid chromatography (LC), high performance liquid chromatography (HPLC), gas chromatography (GC), thin film chromatography, size exclusion chromatography, or affinity chromatography may be used.

In addition, in the isolation process, conditions for isolating 11-dehydro TXB₂, tetranor-PGFM, tetranor PGDM, and tetranor PGEM among the above-described biomarkers from a urine sample are familiar, and therefore, in the measurement using mass spectrometry, preferably, 11-dehydro TXB₂, tetranor-PGFM, tetranor PGDM, or tetranor PGEM, and more preferably, 11-dehydro TXB₂, tetranor-PGFM, tetranor PGDM, and tetranor PGEM are used as a target biomarker.

Examples of mass spectrometry used in the measurement step may include gas chromatography mass spectrometry (GC-MS), liquid chromatography mass spectrometry (LC-MS), direct-injection mass spectrometry or Fourier transform ion cyclotron resonance mass spectrometry (FT-ICR-MS), capillary electrophoresis mass spectrometry (CE-MS), high performance liquid chromatography-coupled mass spectrometry (HPLC-MS), quadrupole mass spectrometry, tandem mass spectrometry coupled with the above-described isolation method (e.g., LC-MS/MS, HPLC-MS/MS, etc.), inductively coupled plasma mass spectrometry (ICP-MS), pyrolysis mass spectroscopy (Py-MS), ion mobility mass spectrometry, and time-of-flight mass spectrometry (TOF MS). Among these, as the mass spectrometry, LC-MS or tandem mass spectrometry coupled with the above-described isolation method is preferably used.

When mass spectrometry is used in the measurement step, to quantify the content of the above-described biomarker in a urine sample, a pretreatment step of adding an internal standard to the urine sample may be provided. Examples of the internal standard may include deuterated LTC₄, deuterated 6-keto-PGF_{1α}, deuterated tetranor-PGDM, deuterated tetranor-PGEM, and deuterated PGE₂, which may be used alone or in combination thereof.

### [Evaluation step]

In the testing method of the exemplary embodiment, following the above-described [measurement step], based on the content of the above-described biomarker in a urine sample, an evaluation step of evaluating the risk of developing a food allergy and severity may be included.

In the evaluation step, when the content of, among the above-described biomarkers, at least one selected form the group consisting of LTE₄, 14,15-LTE₄, 11-trans LTE₄, 11-dehydro TXB₂, 2,3-dinor-8-iso PGF_{2α}, 13,14-dihydro-15-keto-tetranor PGF_{1β}, 6,15-diketo-13,14-dihydro PGF_{1α}, tetranor-PGFM, 20-OH PGE₂, 13,14-dihydro-15-keto-tetranor PGD₂ and 13,14-dihydro-15-keto-tetranor PGE₂ in a urine sample is higher than others, it can be determined that the symptoms of a food allergy are severe or more severe, or the risk of developing a food allergy is high or higher.

On the other hand, when, among the above-described biomarkers, the PGE₃ or PGD₃ content in a urine sample is lower, it can be determined that the symptoms of a food allergy are severe or more severe, or the risk of developing a food allergy is high or higher.

The content of the above-described biomarker in a urine sample may be determined comparing the contents of the above-described biomarker in urine samples collected multiple times from the same subject. At this time, changes in the severity and the risk of developing a food allergy in the same subject may be evaluated. In addition, whether the content of the biomarker in a urine sample is higher or not may be determined by comparing the contents of the biomarker in urine samples collected from a plurality of subjects. At this time, whether the severity and the risk of developing a food allergy for one subject are high or not may be evaluated by comparison with other subjects.

Further, in the evaluation process, when, among the above-described biomarkers, the content of at least one selected from the group consisting of LTE₄, 14,15-LTE₄, 11-trans LTE₄, 11-dehydro TXB₂, 2,3-dinor-8-iso PGF_{2α}, 13,14-dihydro-15-keto-tetranor PGF_{1β}, 6,15-diketo-13,14-dihydro PGF_{1α}, tetranor-PGFM, 20-OH PGE₂, 13,14-dihydro-15-keto-tetranor PGD₂ and 13,14-dihydro-15-keto-tetranor PGE₂ in a urine sample is higher than a predetermined value, it can be determined that symptoms of a food allergy is severe or more severe, or the risk of developing a food allergy is high or higher. On the other hand, when the content mentioned above is lower than the predetermined value, it can be determined that symptoms of food allergy are mild or milder, or the risk of developing a food allergy is low or lower.

Furthermore, when, among the above-described biomarkers, the content of PGE₃ or PGD₃ in a urine sample is lower than the predetermined value, it can be determined that symptoms of a food allergy is severe or more severe, or the risk of developing a food allergy is high or higher. However, when the content mentioned above is higher than the predetermined value, it can be determined that symptoms of food allergy are mild or milder, or the risk of developing a food allergy is low or lower.

The predetermined value (cut-off value) may be determined according to a conventional method by comparing the contents of the above-described biomarker in urine samples of a plurality of healthy persons or non-food allergy patients with those in urine samples of a plurality of food allergy patients.

In addition, by determining not only the contents of the above-described 13 types of biomarkers but also the content of tetranor-PGDM or tetranor-PGEM in a urine sample, the symptoms of a food allergy and the risk of developing a food allergy can be evaluated with a higher accuracy. More specifically, when the tetranor-PGDM content in urine is higher than the predetermined value, it is determined that the symptoms are severe and the risk of developing a food allergy is high, and when the tetranor-PGDM content in urine is lower than the predetermined value, it can be determined that the symptoms are mild and the risk of developing a food allergy is low. In addition, when an increase in the tetranor-PGDM content in urine is maintained for a predetermined period, and the tetranor-PGEM in urine is quickly declined as soon as it transiently increases, it can be determined as food allergy. On the other hand, when there is no change in the tetranor-PGDM in urine, and the tetranor-PGEM content in urine is consistently high, it can be seen that an inflammatory disease, other than a food allergy, occurs.

In the evaluation step, the activation of mast cells in a food allergy may also be evaluated.

The "activation of mast cells" used herein refers to an increase in the number of mast cells, an increase in degranulation and an increase in production amount of an active substance. The content of the above-described biomarker in a urine sample is increased or decreased according to the increase in mast cells, and is decreased or increased while the degranulation is suppressed. Therefore, the activation of mast cells can be evaluated using the content of the above-described biomarker in a urine sample as an indicator.

### [Usage]

The testing method of the exemplary embodiment may be used in evaluation of a drug administered to a food allergy patient, or a method for treating the patient. For example, when the contents of the above-described biomarker in urine samples collected are measured at arbitrary points of time from before the initiation of treatment to after the initiation of treatment, if the contents of the above-described biomarker are decreased or increased, it can be determined that the drug or the method is effective. On the other hand, when the content of the above-described biomarker is not decreased but increased, or not increased but decreased, it can be determined that the drug or method is not effective. At this time, a decrease or increase in the content of the above-described biomarker may not be significant, and may be determined to be likely to increase or decrease by those or ordinary skill in the art.

As the method for treating a patient, for example, hyposensitization therapy may be used. The administration route, dose and frequency for hyposensitization therapy are not particularly limited, and may be suitably selected according to various conditions such as a patient's age, body weight, symptoms, etc. Examples of the administration route to a subject may include intradermal, subcutaneous, intramuscular, intraperitoneal, transdermal, transmucosal, oral administration and inhalation.

The testing method of the exemplary embodiment may also be used in an animal experiment for developing a therapeutic drug for a food allergy to evaluate the effect of the drug.

The testing method of the exemplary embodiment may also be used in examination of a sample collected from a subject to obtain information necessary for diagnosis. Such a testing method may be carried out, for example, by a testing agency.

### <Kit for testing urine sample>

In one embodiment, the present invention provides a kit for testing a urine sample for a food allergy, which includes at least one selected from the group consisting of an anti-LTE₄ antibody, an anti-14,15-LTE₄ antibody, an anti-11-trans LTE₄ antibody, an anti-11-dehydro TXB₂ antibody, an anti-2,3-dinor-8-iso PGF_{2α} antibody, an anti-13,14-dihydro-15-keto-tetranor PGF_{1β} antibody, an anti-6,15-diketo-13,14-dihydro PGF_{1α} antibody, an anti-tetranor-PGFM antibody, an anti-20-OH PGE₂ antibody, an anti-PGE₃ antibody, an anti-PGD₃ antibody, an anti-13,14-dihydro-15-keto-tetranor PGD₂ antibody and an anti-13,14-dihydro-15-keto-tetranor PGE₂ antibody (an "antibody specifically binding to the above-described biomarker" is a generic term for the 13 types of antibodies).

The kit for testing a urine sample of the exemplary embodiment may be used to investigate a food allergy using the above-described testing method, and may facilitate testing for children through the elderly simply at home.

The kit for testing a urine sample of the exemplary embodiment may also include an anti-tetranor-PGDM antibody or an anti-tetranor-PGEM antibody. Using the kit for testing a urine sample of the exemplary embodiment, by measuring a tetranor PGDM or tetranor PGEM content in addition of at least one of the above-described 13-types of lipids, a plurality of the lipids can be used as indicators, and therefore it is possible to evaluate the presence or absence of a food allergy, the risk of developing a food allergy and severity with a higher accuracy.

The kit for testing a urine sample of the exemplary embodiment may be used for immunoassay using an antibody specifically binding to the above-described biomarker, and may include a reagent and an apparatus, which are required for measuring the content of the above-described biomarker.

### [Measurement according to sandwich method (1)]

When the content of the above-described biomarker, for example, LTE₄ is measured by a sandwich method, the kit for testing a urine sample of the exemplary embodiment may include: a microtiter plate; an anti-LTE₄ antibody for capturing an antigen (LTE₄) (hereinafter, referred to as "capturing antibody 1"); an anti-LTE₄ antibody labeled with a peroxidase or alkaline phosphatase (hereinafter, referred to as "labeled antibody 1"); and a peroxidase substrate (e.g., DAB, TMB, OPD, etc.) or an alkaline phosphatase substrate (e.g., NPP, etc.).

The capturing antibody 1 and the labeled antibody 1 may serve to recognize different epitopes of the antigen (LTE₄).

Usages of the kit for testing a urine sample will be described below. First, capturing antibody 1 is immobilized onto a microtiter plate. Subsequently, a urine sample which is suitably diluted is added to the plate and incubated. Subsequently, the urine sample is removed and washed. Subsequently, labeled antibody 1 is added and incubated. Afterward, a substrate is added and developed. Luminescence is detected using a microtiter plate reader, thereby calculating the LTE₄ content.

Even using an antibody that specifically binds to a biomarker, other than LTE₄, the content of the biomarker may be determined by the same method as described above.

### [Measurement by sandwich method (2)]

When the content of the above-described biomarker, for example, LTE₄ is measured by a sandwich method using a secondary antibody, the kit for testing a urine sample of the exemplary embodiment may include: a microtiter plate; an anti-LTE₄ antibody for capturing an antigen (LTE₄) (hereinafter, referred to as "capturing antibody 2"); an anti-LTE₄ antibody (hereinafter, referred to as "primary antibody 1") as a primary antibody; an antibody against an anti-LTE₄ antibody labeled with a peroxidase or alkaline phosphatase (hereinafter, referred to as "secondary antibody 1") as a secondary antibody; and a peroxidase substrate (e.g., DAB, TMB, OPD, etc.) or an alkaline phosphatase substrate (e.g., NPP, etc.).

The capturing antibody 2 and the primary antibody 1 are preferably act to recognize different epitopes of the antigen (LTE₄)

Usages of the kit for testing a urine sample will be described below. First, capturing antibody 2 is immobilized onto a microtiter plate. Subsequently, a urine sample which is suitably diluted is added to the plate and incubated. Subsequently, the urine sample is removed and washed. Subsequently, primary antibody 1 is added and incubated, and then the plate is washed. Subsequently, secondary antibody 1 is added and incubated. Then, a substrate is added to perform development. Luminescence was detected using a microtiter plate reader, thereby obtaining an LTE₄ content. In addition, due to the use of the secondary antibody 1, the reaction is amplified and thus detection sensitivity maybe enhanced.

Even using an antibody that specifically binds to a biomarker, other than LTE₄, the content of the biomarker may also be calculated by the same method as described above.

The above-described labeled antibody 1 and the above-described secondary antibody 1 are not limited to enzyme-labeled antibodies, and may be antibodies labeled with a radioactive substance such as ¹²⁵I, ¹³¹I, ³⁵S, ³H or the like, a fluorescent substance such as fluorescein isothiocyanate, rhodamine, dansyl chloride, phycoerythrin, tetramethylrhodamine isothiocyanate, a near-infrared fluorescent substance or the like, a luminescent substance such as luciferase, luciferin, aequorin or the like, or nanoparticles such as gold colloids, quantum dots or the like.

In addition, the above-described labeled antibody 1 and the above-described secondary antibody 1 may be biotinylated antibodies, and in this case, the kit for testing a urine sample of the exemplary embodiment may include labeled avidin or streptavidin.

### [Measurement using mass spectrometry]

When the above-described biomarker is measured by mass spectrometry, the kit for testing a urine sample of the exemplary embodiment may include at least one selected from the group consisting of deuterated LTC₄, deuterated 6-keto-PGF_{1α}, deuterated tetranor-PGEM, and deuterated PGE₂ as an internal standard. By using these as internal standards, when the content of the above-described biomarker in a urine sample is measured, extraction efficiency and ionization efficiency for each analysis can be corrected.

As the deuterated LTC₄, for example, LTC₄-d5 may be used. As the deuterated 6-keto-PGF_{1α}, for example, 6-keto-PGF_{1α}-d4 may be used. As the deuterated tetranor-PGEM, for example, tetranor-PGEM-d6 may be used. As the deuterated PGE₂, for example, PGE₂-d4 may be used.

The kit for testing a urine sample of the exemplary embodiment may also include deuterated tetranor-PGDM. As the tetranor PGDM or tetranor PGEM content, in addition to at least one of the above-described 13 types of lipids, is measured using the kit for testing a urine sample of the exemplary embodiment, a plurality of substrates are used as indicators, and therefore the presence or absence of a food allergy, the risk of developing a food allergy and severity can be determined with a higher accuracy.

As the deuterated tetranor-PGDM, for example, tetranor-PGDM-d6 may be used.

The kit for testing a urine sample of the exemplary embodiment may also include a buffer, an enzyme reaction stop solution, and a macroplate reader, which are required for testing.

The kit for testing a urine sample of the exemplary embodiment can be used in evaluation of the activation of mast cells involved in a food allergy.

### <Stick for testing urine sample>

According to an embodiment, the present invention provides a stick for testing a urine sample for a food allergy, which includes at least one selected from the group consisting of an anti-LTE₄ antibody, an anti-14,15-LTE₄ antibody, an anti-11-trans LTE₄ antibody, an anti-11-dehydro TXB₂ antibody, an anti-2,3-dinor-8-iso PGF_{2α} antibody, an anti-13,14-dihydro-15-keto-tetranor PGF_{1β} antibody, an anti-6,15-diketo-13,14-dihydro PGF_{1α} antibody, an anti-tetranor-PGFM antibody, an anti-20-OH PGE₂ antibody, an anti-PGE₃ antibody, an anti-PGD₃ antibody, an anti-13,14-dihydro-15-keto-tetranor PGD₂ antibody and an anti-13,14-dihydro-15-keto-tetranor PGE₂ antibody.

The stick for testing a urine sample of the exemplary embodiment may be used to perform examination of a food allergy using the above-described testing method, and may facilitate testing simply at home with respect to children through the elderly.

The stick for testing a urine sample of the exemplary embodiment is a stick-type testing agent, for example, which is able to visualize the content of the above-described biomarker in a urine sample by a colored line or the like.

The stick for testing a urine sample of the exemplary embodiment may also include an anti-tetranor-PGDM antibody or an anti-tetranor-PGEM antibody. As the tetranor PGDM or tetranor PGEM content, in addition to at least one of the above-described 13 types of lipids, is measured using the kit for testing a urine sample of the exemplary embodiment, a plurality of substrates are used as indicators, and therefore the presence or absence of a food allergy, the risk of developing a food allergy and severity can bae determined with a higher accuracy.

As the stick for testing a urine sample, one that is known in the art can be used, or for example, one that is configured for use in immunochromatography can be used.

When the content of the above-described biomarker, for example, LTE₄, is measured by immunochromatography, the stick for testing a urine sample of the exemplary embodiment may be formed by connecting an antibody-containing unit for containing an anti-LTE₄ antibody, which is labeled with gold colloids or the like, with a determining unit in which a secondary anti-LTE₄ antibody recognizing a different epitope of LTE₄ is linearly immobilized onto a cellulose membrane or the like through a thin groove.

Usages of the stick for testing a urine sample will be described below. A urine sample is added to the stick for testing a urine sample, and an antigen-antibody reaction between a primary anti-LTE₄ antibody and LTE₄ occurs in the antibody-containing unit, thereby forming a LTE₄-primary anti-LTE₄ antibody complex. Subsequently, due to a capillary phenomenon, the complex is transferred to the determining unit through the groove. Subsequently, the complex is captured by a secondary anti-LTE₄ antibody immobilized onto the determining unit. Subsequently, due to the plasmon effect of the gold colloids, a red line is expressed in the determining unit, and thereby LTE₄ can be detected.

Even using an antibody that specifically binds to a biomarker, other than LTE₄, the biomarker can be detected by the same method as described above.

The stick for testing a urine sample of the exemplary embodiment may be used to evaluate the activation of mast cells involved in a food allergy.

Hereinafter, the present invention will be described with reference to examples, but the present invention is not limited to the following examples.

### Examples

### [Example 1] Dynamics of lipid mediators in urine samples of mouse models with food allergy

### (1) Construction of ovalbumin (OVA)-inducible mouse models with food allergy

### (1-1) Sensitizing conditions

50 µg of OVA and 1 mg of alum were intraperitoneally administered to wild-type BALB/c mice (6 to 12-week-old, female), and two weeks later, 50 µg of OVA was intraperitoneally administered to sensitize the mice.

### (1-2) Stimulation conditions

Subsequently, two weeks after the second administration, 10 mg of OVA was orally administered to the mice every other day for a total of 10 times.

### (1-3) Urine sampling

Subsequently, during observation following the 10^{th} stimulation, urine was collected from each of the mice (n=5). As controls, during observation, urine was collected from untreated mice (n=3).

### (2) Pretreatment of urine samples

As internal standards, LTC₄-d5, 6-keto-PGF_{1α}-d4, tetranor-PGDM-d6, tetranor-PGEM-d6, or PGE₂-d4 was added to urine supernatants. Subsequently, using hydrochloric acid and ethanol, urine samples were prepared to be 50% ethanol-containing solutions of pH 3 to 4. Subsequently, the prepared urine samples were injected into a Sep-Pak C18 cartridge (produced by Waters Corporation) equilibrated with ethanol and pure water. Subsequently, contaminants were washed with 3 mL of water and 3 mL of ×2 hexane. Subsequently, a desired component was eluted with 0.05% methanol-formic acid. Afterward, following drying under reduced pressure for 5 hours, the eluted component was redissolved in 100% methanol and filtered.

### (3) Measurement of lipid mediators

The pretreated urine samples obtained in (2) were injected into a triple quadrupole mass spectrometer (LC-MS8030, produced by Shimadzu Corporation) equipped with an electrospray ionization source, and comprehensive lipid analysis was performed using the software "LC/MS/MS method package (Shimadzu Corporation)". As a result, when the content of a lipid in each of the urine samples of the untreated mice was set to be 1, graphs showing relative values of the contents of lipids in the urine samples of the mouse models with food allergy are shown in FIGS. 1A to 1I. Referring to FIGS. 1A to 1I, the "naive" represents an untreated mouse, and the "OVA×10" represents a mouse model with food allergy.

### (4) Result

Referring to FIGS. 1A to 1I, it is shown that, compared with the control groups, in the urine samples of the OVA-induced mouse models with food allergy, five components, that is, 13,14-dihydro-15-keto-tetranor PGF_{1β}, 13,14-dihydro-15-keto-tetranor PGD₂, 13,14-dihydro-15-keto-tetranor PGE₂, 6,15-diketo-13,14-dihydro PGF_{1α}, and 20-OH PGE₂ are significantly increased. In addition, it was shown that the two components, that is, PGD₃ and PGE₃, are significantly decreased.

### [Example 2] Dynamics of lipid mediators in urine samples of patients (humans) for food allergy intolerance test

### (1) Acquisition of urine samples

Food intolerance tests were conducted on food allergy patients (males and females) aged 3 to 22 years at suitable intake amounts and intervals under the supervision of a doctor, and then the patients were rated from 0 to 2 according to the degree of symptoms. In addition, a higher score represents a patient with more severe symptoms. From each of the patients with scores of 0 and 2, urine was collected before the intolerance test (hereinafter, referred to as "score 0 pre" or "score 2 pre") and after the intolerance test (hereinafter, referred to as "score 0 post" or "score 2 post"). In addition, the number of patients with "score 0 pre" is 9, the number of patients with "score 2 pre" is 10, the number of patients with "score 0 post" is 10, and the number of patients with "score 2 post" is 10.

### (2) Pretreatment of urine samples

The urine samples were pretreated by the same method as described in Example 1 (2), except that TXM-d4 was additionally used as an internal standard.

### (3) Measurement of lipid mediators

Comprehensive lipid analysis was performed on the urine samples undergoing the pretreatment described in (2) by the same method as described in Example 1 (3). As a result, graphs showing relative values of the contents of lipids in the urine samples of the patients with a score of 0 after the tolerance test and the patients with a score of 2 before and after the tolerance test, when the content of each lipid in each of the urine samples of the patients with a score of 0 before the tolerance test was set to be 1, are shown in FIGS. 2A to 2J.

### (4) Results

Referring to FIGS. 2A to 2J, it was shown that, compared with score 0 post, in score 2 post, 6 components, that is, tetranor-PGFM, 13,14-dihydro-15-keto-tetranor PGF_{1β}, 11-dehydro TXB₂, 2,3-dinor-8-iso PGF_{2α}, 14,15-LTE₄ and 11-trans LTE₄, are significantly increased. In addition, it was shown that, compared with score 2 pre, in score 2 post, five components, that is, 13,14-dihydro-15-keto-tetranor PGF_{1β}, 11-dehydro TXB₂, 2,3-dinor-8-iso PGF_{2α}, 13,14-dihydro-15-keto-tetranor PGD₂ and 13,14-dihydro-15-keto-tetranor PGE₂, are significantly increased.

In addition, referring to FIG. 2C, compared with score 0 pre and score 0 post, in score 2 pre, tetranor-PGFM is increased, which indicates that there is a likelihood of the use of tetranor-PGFM as a marker that reflects the innate characteristics of a food allergy.

According to Examples 1 and 2, it can be seen that, in mice and humans, all of 13,14-dihydro-15-keto-tetranor PGFip, 13,14-dihydro-15-keto-tetranor PGD₂ and 13,14-dihydro-15-keto-tetranor PGE₂ are significantly increased.

On the other hand, 6,15-diketo-13,14-dihydro PGF_{1α} and 20-OH PGE₂ were significantly increased only in mice, whereas PGD₃ and PGE₃ were significantly decreased only in mice.

Therefore, it was demonstrated that the above-mentioned four components are effectively used as biomarkers in an experimental system for evaluating food allergenicity using mice.

In addition, tetranor-PGFM, 2,3-dinor-8-iso PGF_{2α}, 11-dehydro TXB₂, 14,15-LTE₄, and 11-trans LTE₄ were significantly increased only in humans.

Therefore, it was demonstrated that the above-mentioned five components are effectively used as biomarkers for suitably evaluating the risk of developing a food allergy and severity in humans, or for evaluating responsiveness to hyposensitization therapy.

### [Example 3] Dynamics of lipid mediators in urine samples of patients (humans) for food allergy intolerance test 2

### (1) Acquisition of urine samples

Food intolerance tests were conducted on food allergy patients (males and females) aged 3 to 22 years at suitable intake amounts and intervals under the supervision of a doctor, and then the patients were rated from 0 to 4 according to the degree of symptoms. In addition, a higher score represents a patient with more severe symptoms. From each of the patients with scores of 0 and 4, urine was collected before the intolerance test (hereinafter, referred to as "score 0 pre" or "score 4 pre") and after the intolerance test (hereinafter, referred to as "score 0 post" or "score 4 post"). In addition, the number of patients with "score 0 pre" is 8, the number of patients with "score 4 pre" is 10, the number of patients with "score 0 post" is 8, and the number of patients with "score 4 post" is 10.

### (2) Pretreatment of urine samples

As internal standards, tetranor-PGDM-d6, tetranor-PGEM-d6, TMX-d4, and LTC₄-d5 were added to urine supernatants. Subsequently, using hydrochloric acid, urine samples were prepared to have pH 3 to 4. Subsequently, the prepared urine samples were injected into a Sep-Pak C18 cartridge (produced by Waters Corporation) equilibrated with ethanol and pure water. Subsequently, contaminants were washed with 3 mL of water and 3 mL of ×2 hexane. Subsequently, a desired component was eluted with methanol. Afterward, following drying under reduced pressure for 4 hours, the eluted component was redissolved in 80% methanol and filtered.

### (3) Measurement of lipid mediators

The pretreated urine samples obtained in (2) were injected into a triple quadrupole mass spectrometer (LC-MS8030, produced by Shimadzu Corporation) equipped with an electrospray ionization source, and comprehensive lipid analysis was performed using the software "LabSolutions (Shimadzu Corporation)". As a result, graphs showing the content (absolute value) of a lipid in each of the urine samples of the patients with scores 0 and 4 before and after the intolerance test are shown in FIGS. 3A to 3D. In addition, referring to FIGS. 3A to 3D, the units of the vertical axis "ng/mg Cre" represents the content (ng) of a lipid relative to 1 mg of creatine in the urine sample.

### (4) Result

Referring to FIGS. 3A to 3D, it is shown that, compared with score 0 post, in score 4 post, tetranor-PGDM, tetranor-PGEM, 11-dehydro TXB₂, and LTE₄ are significantly increased. In addition, compared with score 4 pres, in score 4 post, tetranor-PGDM, tetranor-PGEM, 11-dehydro TXB₂ and LTE₄ are significantly increased.

As described above, it was demonstrated that all four components, which include the two components, that is, 11-dehydro TXB₂ and LTE₄, which are first identified in the present invention, and the known two components, that is, tetranor-PGDM and tetranor-PGEM, are effectively used as biomarkers for humans to suitably evaluate the risk of developing a food allergy and severity, or to evaluate responsiveness to hyposensitization therapy, and by using the four components as indicators, the presence or absence of a food allergy, the risk of developing a food allergy and severity can be determined with a higher accuracy.

### INDUSTRIAL APPLICABILITY

According to the present invention, the presence or absence of a food allergy, the risk of developing a food allergy and severity may be suitably evaluated. In addition, before allergic symptoms are shown, preventive treatment may be performed through risk evaluation. In addition, through the evaluation of the responsiveness to hyposensitization therapy, effective treatment may be carried out by administering an allergen as much as possible within a safe range.

In addition, the testing method of the present invention does not require medical techniques such as blood collection, and thus testing can be performed simply at home with respect to children through the elderly using the kit for testing a urine sample or stick for testing a urine sample according to the present invention.

## Claims

1. A biomarker for testing food allergy, comprising:
at least one selected from the group consisting of Leukotriene E₄ (LTE₄), 14,15-LTE₄, 11-trans LTE₄, 11-dehydro Thromboxane B₂ (11-dehydro TXB₂), 2,3-dinor-8-iso Prostaglandin F_{2α}, 13,14-dihydro-15-keto-tetranor PGF_{1β}, 6,15-diketo-13,14-dihydro PGF_{1α}, tetranor-Prostaglandin F Metabolite (tetranor-PGFM), 20-hydroxy Prostaglandin E₂ (20-OH PGE₂), PGE₃, Prostaglandin D₃ (PGD₃), 13,14-dihydro-15-keto-tetranor PGD₂, and 13,14-dihydro-15-keto-tetranor PGE₂.

2. A food allergy testing method, comprising:
measuring the content of a biomarker in a urine sample of a subject,
wherein the biomarker is at least one selected from the group consisting of LTE₄, 14,15-LTE₄, 11-trans LTE₄, 11-dehydro TXB₂, 2,3-dinor-8-iso Prostaglandin F_{2α}, 13,14-dihydro-15-keto-tetranor PGF_{1β}, 6,15-diketo-13,14-dihydro PGF_{1α}, tetranor-PGFM, 20-OH PGE₂, PGE₃, PGD₃, 13,14-dihydro-15-keto-tetranor PGD₂, and 13,14-dihydro-15-keto-tetranor PGE₂.

3. The method according to claim 2, further comprising:
in the measurement step, measuring the content of tetranor-PGDM or tetranor-PGEM.

4. The method according to claim 2 or 3, further comprising:
an evaluation step of evaluating, as a urine sample contains a high or low biomarker content, symptoms of food allergy are severe or more severe, or the risk of developing a food allergy is high or higher.

5. The method according to any one of claims 2 to 4, which is used to evaluate a method or drug for treating a food allergy.

6. The method according to claim 5, which is used in hyposensitization therapy.

7. The method according to any one of claims 2 to 6, wherein the measurement step is performed by immunoassay or mass spectrometry.

8. The method according to claim 7, further comprising:
a pretreatment step of adding at least one selected from the group consisting of deuterated LTC₄, deuterated 6-keto-PGF_{1α}, deuterated tetranor-PGDM, deuterated tetranor-PGEM, and deuterated PGE₂ as an internal standard to urine samples, and wherein the measurement step is performed by mass spectrometry.

9. The method according to any one of claims 2 to 8, further comprising, in the evaluation step, evaluating the activation of mast cells involved in a food allergy.

10. A kit for testing a urine sample for a food allergy, comprising:
at least one selected from the group consisting of an anti-LTE₄ antibody, an anti-14,15-LTE₄ antibody, an anti-11-trans LTE₄ antibody, an anti-11-dehydro TXB₂ antibody, an anti-2,3-dinor-8-iso PGF_{2α} antibody, an anti-13,14-dihydro-15-keto-tetranor PGFip antibody, an anti-6,15-diketo-13,14-dihydro PGF_{1α} antibody, an anti-tetranor-PGFM antibody, an anti-20-OH PGE₂ antibody, an anti-PGE₃ antibody, an anti-PGD₃ antibody, an anti-13,14-dihydro-15-keto-tetranor PGD₂ antibody, and an anti-13,14-dihydro-15-keto-tetranor PGE₂ antibody.

11. The kit according to claim 10, further comprising:
an anti-tetranor-PGDM antibody or an anti-tetranor-PGEM antibody.

12. The kit according to claim 10 or 11, further comprising:
at least one selected from the group consisting of deuterated LTC₄, deuterated 6-keto-PGF_{1α}, deuterated tetranor-PGEM, and deuterated PGE₂.

13. The kit according to any one of claims 10 to 12, further comprising:
deuterated tetranor-PGDM.

14. A stick for testing a urine sample for food allergy, comprising:
at least one selected from the group consisting of an anti-LTE₄ antibody, an anti-14,15-LTE₄ antibody, an anti-11-trans LTE₄ antibody, an anti-11-dehydro TXB₂ antibody, an anti-2,3-dinor-8-iso PGF_{2α} antibody, an anti-13,14-dihydro-15-keto-tetranor PGFip antibody, an anti-6,15-diketo-13,14-dihydro PGF_{1α} antibody, an anti-tetranor-PGFM antibody, an anti-20-OH PGE₂ antibody, an anti-PGE₃ antibody, an anti-PGD₃ antibody, an anti-13,14-dihydro-15-keto-tetranor PGD₂ antibody, and an anti-13,14-dihydro-15-keto-tetranor PGE₂ antibody.

15. The stick according to claim 14, further comprising:
an anti-tetranor-PGDM antibody or an anti-tetranor-PGEM antibody.
